(19) Europäisches Patentamt / European Patent Office / Office européen des brevets

(11) **EP 3 936 135 A1**

(12) **EUROPEAN PATENT APPLICATION**
published in accordance with Art. 153(4) EPC

(43) Date of publication:
**12.01.2022 Bulletin 2022/02**

(51) Int Cl.:
*A61K 31/7088* *(2006.01)*    *A61K 31/712* *(2006.01)*
*A61K 31/7125* *(2006.01)*    *A61K 48/00* *(2006.01)*
*A61P 21/04* *(2006.01)*    *C07K 7/02* *(2006.01)*
*C07K 14/00* *(2006.01)*    *B82Y 5/00* *(2011.01)*
*B82Y 40/00* *(2011.01)*    *A61K 9/00* *(2006.01)*
*A61K 9/10* *(2006.01)*    *A61K 9/14* *(2006.01)*
*A61K 35/76* *(2015.01)*    *C12N 7/02* *(2006.01)*
*A61K 47/64* *(2017.01)*    *C12N 15/113* *(2010.01)*

(21) Application number: 20748647.3

(22) Date of filing: 29.01.2020

(86) International application number:
**PCT/JP2020/003146**

(87) International publication number:
**WO 2020/158792 (06.08.2020 Gazette 2020/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.01.2019 JP 2019014738**

(71) Applicants:
• **NATIONAL CENTER OF NEUROLOGY AND
PSYCHIATRY
Kodaira-shi
Tokyo 187-8551 (JP)**

• **Nippon Medical School Foundation
Tokyo 113-8602 (JP)**

(72) Inventors:
• **OKADA, Takashi
Tokyo 113-8602 (JP)**
• **KINOH, Hiromi
Tokyo 113-8602 (JP)**
• **AOKI, Yoshitsugu
Kodaira-shi, Tokyo 187-8551 (JP)**
• **TAKEDA, Shin'ichi
Kodaira-shi, Tokyo 187-8551 (JP)**

(74) Representative: **Müller, Christian Stefan Gerd
ZSP Patentanwälte PartG mbB
Hansastraße 32
80686 München (DE)**

(54) **NUCLEIC ACID DELIVERY COMPLEX**

(57) Provided are a complex that comprises a nucleic acid-containing nanoparticle and a hollow particle of a non-enveloped virus, a method for producing the complex, and a pharmaceutical composition comprising the complex.

[FIG. 7]

20 nm

40–100 nm

SEQUENCE LISTING

<110> NATIONAL CENTER OF NEUROLOGY AND PSYCHIATRY
NIPPON MEDICAL SCHOOL FOUNDATION

<120> Nucleic acid delivery complex

<130> 675263

<150> JP2019-014738
<151> 2019-01-30


<160> 5

<210> 1
<211> 22
<212> PRT
<213> Artificial Sequence

<220>
<223> Pip6a peptide

<220>
<221> SITE
<222> (2)..(2)
<223> Xaa is aminohexanoic acid

<220>
<221> MOD_RES
<222> (5)..(5)
<223> bAla

<220>
<221> SITE
<222> (8)..(8)
<223> Xaa is aminohexanoic acid

<220>
<221> SITE
<222> (16)..(16)
<223> Xaa is aminohexanoic acid

<220>
<221> MOD_RES
<222> (18)..(18)
<223> bAla

<220>
<221> SITE
<222> (20)..(20)
<223> Xaa is aminohexanoic acid

<220>
<221> SITE
<222> (22)..(22)
<223> May optionally comprise a linker at the C-terminal end, which may
be one of BCys, XCys, GGCys, BBCys, BXCys or XBCys, X, XX, B, BB,
BX, or XB wherein X is Ahx and B is bAla

<220>
<221> MOD_RES
<222> (22)..(22)

```
<223>  bAla


<400>  1
Arg Xaa Arg Arg Ala Arg Arg Xaa Arg Tyr Gln Phe Leu Ile Arg Xaa
1               5                   10                  15


Arg Ala Arg Xaa Arg Ala
                20


<210>  2
<211>  14
<212>  PRT
<213>  Artificial Sequence

<220>
<223>  B peptide

<220>
<221>  SITE
<222>  (2)..(2)
<223>  Xaa is aminohexanoic acid

<220>
<221>  MOD_RES
<222>  (5)..(5)
<223>  bAla

<220>
<221>  SITE
<222>  (8)..(8)
<223>  Xaa is aminohexanoic acid

<220>
<221>  MOD_RES
<222>  (11)..(11)
<223>  bAla

<220>
<221>  SITE
<222>  (13)..(13)
<223>  Xaa is aminohexanoic acid

<220>
<221>  SITE
<222>  (14)..(14)
<223>  May optionally comprise a linker at the C-terminal end, which may
       be one of BCys, XCys, GGCys, BBCys, BXCys or XBCys, X, XX, B, BB,
       BX, or XB wherein X is Ahx and B is bAla

<220>
<221>  MOD_RES
<222>  (14)..(14)
<223>  bAla


<400>  2
Arg Xaa Arg Arg Ala Arg Arg Xaa Arg Arg Ala Arg Xaa Ala
1               5                   10
```

```
<210>    3
<211>    25
<212>    DNA
<213>    Mus musculus

<400>    3
ttgttttatc cataccttct gtttg                                        25


<210>    4
<211>    20
<212>    DNA
<213>    Artificial Sequence

<220>
<223>    Ex50F primer

<400>    4
gagtgggagg ctgtaaacca                                              20


<210>    5
<211>    20
<212>    DNA
<213>    Artificial Sequence

<220>
<223>    Ex53R primer

<400>    5
acctgttcgg cttcttcctt                                              20
```

**Description**

Technical Field

The present invention relates to a complex for delivering a nucleic acid, a method for producing the complex, and a pharmaceutical composition comprising the complex as an active ingredient.

Background Art

Since antisense nucleic acids were reported in the latter half of the 1970s, lots of studies and developments have been carried out aiming at application of nucleic acid molecules as drugs. Especially, the discovery of small interfering RNA (siRNA) that induces RNA interference in mammalian cells, which was reported in the early 2000s, has accelerated the development of nucleic acid drugs. In recent years, the biological functions and importance of non-coding RNAs such as microRNA (miRNA) and long non-coding RNA (lncRNA) have been found. Thus the non-coding RNAs have attracted attention as a new target for nucleic acid drugs. Further, miRNA itself has attracted attention as a nucleic acid drug.

For example, Duchenne muscular dystrophy (DMD) is a serious hereditary disease that exhibits progressive muscle weakness and muscular atrophy, and is often caused by a frameshift due to a deletion mutation in a dystrophin gene. Currently, development of a technique for correcting an amino acid reading frame moved due to the frameshift mutation by exon skipping using an antisense morpholino nucleic acid is underway using DMD model animals (Non-Patent Literature 1).

To date, nucleic acid drugs have had the problem of in vivo degradation. Then, modified nucleic acid technology and drug delivery system (DDS) technology have been remarkably developed, and stable and highly effective candidate products have been developed one after another. For example, DDS prepared by introducing a nucleic acid into the interior of a non-viral hollow particle of adeno-associated virus (AAV) has been established as a technique with high safety and organ tropism (Patent Literature 1).

Furthermore, in order to enhance the therapeutic effects of nucleic acid drugs on target organs and improve the safety and costs of nucleic acid drugs, peptide-conjugated morpholino having high cell membrane permeability has been developed (Non-Patent Literature 2). However, there is a need for establishment of an epoch-making DDS having higher safety, organ/tissue specificity, and high therapeutic effect.

Citation List

Patent Literatures

Patent Literature 1: WO2012/144446

Non-Patent Literatures

Non-Patent Literature 1: Komaki H. et al., Science translational medicine, vol. 10, Issue 4, 37, eaan0713, 2018
Non-Patent Literature 2: Ezzat K et al., NANO LETTERS, vol. 15, pp. 4364, 2015

Summary of Invention

Problem to be solved by the Invention

An object of the present invention is to establish a DDS having high safety, organ/tissue specificity, and high therapeutic effect.

Solutions to the Problems

As a result of diligent research, the present inventors utilized hollow particles generated when preparing non-enveloped viruses as a carrier for nucleic acid drugs, and thereby successfully produced a complex of nucleic acid drug-containing nanoparticles and the hollow particles, wherein the nanoparticles and the hollow particles are electrostatically conjugated with each other. The complex was found to be a DDS having safety, organ/tissue specificity, and high therapeutic effect. Based on these findings and results, the present invention was completed.

The present invention provides the followings.

[1] A complex comprising a nucleic acid-containing nanoparticle and a hollow particle of a non-enveloped virus.

[2] The complex according to [1], wherein the nucleic acid is a nucleic acid derivative selected from the group consisting of a phosphorodiamidate morpholino oligomer (PMO), a peptide-conjugated PMO (P-PMO), a tricyclo DNA (tcDNA), and a 2'O methyl oligomer (2'OMe).

[3] The complex according to [1] or [2], wherein the nucleic acid derivative is a P-PMO containing a peptide having a sequence set forth in SEQ ID NO: 1 or SEQ ID NO: 2.

[4] The complex according to any one of [1] to [3], wherein the complex has the longest axis of 50 to 1000 nm as measured by a transmission electron microscope (TEM).

[5] The complex according to any one of [1] to [4], wherein the hollow particle is a hollow particle of an adeno-associated virus.

[6] The complex according to any one of [1] to [5], wherein the nucleic acid is an antisense nucleic acid of a dystrophin gene.

[7] The complex according to any one of [1] to [6], wherein the nanoparticle is formed by assembly of the nucleic acids.

[8] A method for producing a complex comprising a nucleic acid-containing nanoparticle and a capsid virus, the method comprising:

(i) a step of producing a nanoparticle containing a nucleic acid,
(ii) a step of producing a hollow particle of a non-enveloped virus, and
(iii) a step of mixing the nanoparticle obtained by (i) and the non-enveloped virus hollow particle obtained by (ii).

[9] The method according to [8], wherein the nucleic acid and the hollow particle are mixed at a ratio of 150 to 1500 moles of the nucleic acid to 1 mole of the hollow particle in step (iii).

[10] A pharmaceutical composition comprising the complex according to any one of [1] to [7].

[11] The pharmaceutical composition according to [10], for use in the treatment of Duchenne muscular dystrophy (DMD).

[12] The pharmaceutical composition according to [10] or [11], for systemic intravenous administration.

[13] The pharmaceutical composition according to [10] or [11], for systemic administration.

[14] A method for preventing or treating a disease, the method comprising administering the complex according to any one of [1] to [7] to a subject.

[15] The method according to [14], for the treatment of Duchenne muscular dystrophy (DMD).

[16] The method according to [14] or [15], wherein the complex is systemically administered to the subject.

[17] The method according to [16], wherein the complex is administered systemically intravenously to the subject.

[18] Use of the complex according to any one of [1] to [7] as a DDS.

[19] The use according to [18], wherein the DDS is for systemic administration.

[20] The use according to [19], wherein the DDS is for systemic intravenous administration.

[21] Use of a hollow particle of non-enveloped virus as a carrier for a nucleic acid drug.

[22] The use according to [21], wherein the nucleic acid drug comprises a nucleic acid derivative selected from the group consisting of a phosphorodiamidate morpholino oligomer (PMO), a peptide-conjugated PMO (P-PMO), a tricyclo DNA (tcDNA), and a 2'O methyl oligomer (2'OMe).

[23] The use according to [22], wherein the nucleic acid derivative is a P-PMO containing a peptide having a sequence set forth in SEQ ID NO: 1 or SEQ ID NO: 2.

[24] The use according to any one of [21] to [23], wherein the nucleic acid drug and the hollow particle form a complex and the complex has the longest axis of 50 to 1000 nm.

[25] The use according to any one of [21] to [24], wherein the hollow particle is a hollow particle of adeno-associated virus.

[26] The use according to any one of [21] to [25], wherein the nucleic acid drug comprises an antisense nucleic acid of a dystrophin gene.

[27] The use according to any one of [21] to [26], wherein the nucleic acid drug comprises a nanoparticle formed by assembly of nucleic acids.

[28] The use according to any one of [21] to [27], wherein the nucleic acid drug is systemically administered.

[29] The use according to [28], wherein the nucleic acid drug is systemically intravenously administered.

Effect of the Invention

The present invention provides a DDS having high safety, organ/tissue specificity, and high therapeutic effect.

Brief Description of Drawings

[FIG. 1] Figure 1 shows particle size distributions of the complex of the present invention, AAV hollow particles and P-PMO obtained by DLS (dynamic light scattering) method.
[FIG. 2] Figure 2 shows gel shift assay results of the complex of the present invention and P-PMO.
[FIG. 3] Figure 3 shows efficiency of exon51 skipping induced by the complex of the present invention or P-PMO.
[FIG. 4] Figure 4 shows the number of dystrophin-positive muscle fibers and fluorescence intensity in the muscles of mice to which the complex of the present invention or P-PMO was administered.
[Fig. 5] Figure 5 shows the amount of dystrophin in the muscles of mice to which the complex of the present invention or P-PMO was administered.
[FIG. 6] Figure 6 shows the amount of dystrophin in various muscles of mice to which the complex of the present invention or P-PMO was administered.
[FIG. 7] Figure 7 is a 2D schematic diagram showing an example of the complex of the present invention.

Mode for Carrying out the Invention

(1) Complex of the present invention

The complex of the present invention is a complex comprising a nanoparticle containing a nucleic acid, and a hollow particle of non-enveloped virus.
As used herein, the "nucleic acid" includes a natural nucleic acid, a nucleic acid derivative, and a combination thereof. The "natural nucleic acid" refers to DNA and RNA consisting of only natural nucleotides, which are naturally occurring, connected to each other. The natural nucleic acid as used in embodiments of the present invention is a nucleic acid exogenous to the non-enveloped virus from which the hollow particle constituting the complex is derived and a living organism to which the complex is administered.
The nucleic acid derivative includes a chemically modified nucleic acid, a nucleic acid analog, an artificial nucleic acid, and a combination thereof. The "chemically modified nucleic acid" refers to an artificially chemically modified nucleic acid. Examples thereof include methylphosphonate DNA/RNA, phosphorothioate DNA/RNA, phosphoramidate DNA/RNA, and 2'-O-methyl DNA/RNA. The "nucleic acid analog" refers to an artificially constructed high molecular compound having a structure and/or properties similar to those of a natural nucleic acid. Examples thereof include a peptide nucleic acid (PNA), a peptide nucleic acid having a phosphate group (PHONA), a bridged nucleic acid and/or a locked nucleic acid (BNA/LNA), and a morpholino nucleic acid (including phosphorodiamidate morpholino oligomer: PMO). The morpholino oligomer refers to an oligomer obtained by polymerization of morpholino subunits (monomers). The morpholino subunit has a structure in which the entire ribose (constituent sugar) of a ribonucleotide, which is a constituent unit of RNA, is replaced by a morpholino ring. The "artificial nucleic acid" refers to an artificially produced nucleic acid that does not exist in nature, and includes a nucleic acid comprising a non-natural nucleotide(s) as a part of a natural nucleic acid and a nucleic acid consisting of only non-natural nucleotides connected to each other. As used herein, the "non-natural nucleotide" refers to an artificially constructed or artificially chemically modified nucleotide that does not exist in nature and has a structure and/or properties similar to those of the natural nucleotide. The non-natural nucleotide includes those corresponding to the chemically modified nucleic acid and the nucleic acid analog as described above.
In the nucleic acid used in the present invention, its phosphate group, sugar and/or base may be labeled if necessary. As the label, a labeling substance known in the art can be used. Examples of the labeling substance include radioisotopes (for example, $^{32}P$, $^{3}H$, $^{14}C$), DIG, biotin, fluorescent dyes (for example, FITC, Texas, cy3, cy5, cy7, FAM, HEX, VIC, JOE, Rox, TET, Bodypy 493, NBD, TAMRA), and luminescent substances (for example, acridinium ester).
Examples of the nucleic acid used in the present invention include any gene, mRNA or a fragment thereof, and a nucleic acid having a sequence complementary to the gene, mRNA or fragment thereof, for example, an oligonucleotide. The nucleic acid may be a nucleic acid having a particular biological function, for example, an enzymatic function, a catalytic function, or a biological inhibitory or enhancing function (for example, function of inhibiting or enhancing transcription or translation) in vivo or in a cell, preferably in a cell. Such a nucleic acid is also called a functional nucleic acid. Specific examples of the functional nucleic acid include an RNA interfering agent, a nucleic acid aptamer (including an RNA aptamer and a DNA aptamer), a decoy, an antisense nucleic acid (antisense DNA, antisense RNA, antisense RNA/DNA), a ribozyme (including a deoxyribozyme), a U1 adapter, a molecular beacon, a riboswitch, and a transcription factor binding region. Particularly, the antisense DNA and the RNA interfering agent can be preferably used as the "nucleic acid" in the present invention. As used herein, the "RNA interfering agent" refers to a substance that induces RNA interference (RNAi) in vivo to degrade the transcript of a target gene and thereby suppress (silence) the expression of the target gene. Examples of the RNA interfering agent include siRNA (small interfering RNA), shRNA (short hairpin RNA), and miRNA (micro RNA) (including pri-miRNA and pre-miRNA).

The length of the nucleic acid used in the present invention is not particularly limited, and the nucleic acid may have a length of, for example, 10 to 10,000 nucleotides, preferably 12 to 200 nucleotides, and more preferably 15 to 50 nucleotides.

Nanoparticles generally refer to fine particles with a particle size on the order of nanometers (nm). For the complex of the present invention, a nanoparticle having a diameter of 1 to 1000 nm, preferably a diameter of 10 to 200 nm, and more preferably a diameter of 30 to 90 nm can be used. The nanoparticle comprised in the complex of the present invention may have any shape and any form as long as the whole or partial surface of the nanoparticle has positive charge or negative charge and the nanoparticle contains a nucleic acid. Although the nanoparticle is usually spherical in shape, the nanoparticle can take different forms depending on the property and intended use of the nanoparticle. Examples of the nanoparticle include a liposome, an albumin nanoparticle, a micelle, a dendrimer, a nanoemulsion, a metal nanoparticle, and a copolymer of polylactic acid and polyglycolic acid (PLGA). In the present invention, a nanoparticle containing two or more kinds of functional nucleic acids within the particle can be used.

Liposomes are lipid nanoparticles composed of lipid bilayer membranes. Since cell membranes are mainly composed of phospholipid bilayer membranes, liposomes are excellent in terms of biocompatibility. In the present invention, the nucleic acid is encapsulated inside a liposome, bound to the surface of a liposome, or inserted into the lipid bilayer membrane of a liposome. Various functions can be given to liposomes by modifying the surfaces of the liposomes. For example, liposomes can be modified with PEG to improve the stability in blood. For example, a ligand for a specific receptor or an antibody (or a fragment thereof) against a specific cell (for example, cancer) can be added to the surfaces of liposomes to give target tropism to the liposomes. As used herein, membrane components constituting the liposomes are not particularly limited. Examples of the membrane component include a phospholipid, a glyceroglycolipid, and a glycosphingolipid. Particularly, examples of the phospholipid include DPPC, DSPE-PEG, DSPE-PEG-NHS, EPC, POPC, DSPC, DSPE, PS, PG, PI, DMPG, and DMPC.

Micelles are aggregates of amphipathic molecules having hydrophilic parts and hydrophobic parts. Micelles can have a hydrophobic core or a hydrophilic core. For example, molecules having hydrophilic parts composed of phospholipids and hydrophobic parts composed of fatty acids form a micelle having a hydrophobic core in an aqueous solvent. The micelle may contain polymers. In an embodiment, the micelle may contain homopolymers. Typical examples of the homopolymer include poly(alkylene glycol) [for example, poly(ethylene glycol) (PEG), etc.], poly(amino acid) [for example, poly(aspartic acid), and poly(glutamic acid) (PGA), etc.], poly-(γ-L-glutamylglutamine) (PGGA), poly(phenylene oxide) (PPO), poly(ε-caprolactone) (PCL), and poly(lactic acid). In an embodiment, the micellar carrier may contain poly-(γ-L-glutamylglutamine) (PGGA). In another embodiment, the micelle may contain copolymers, for example, poly(lactic acid-co-glycolic acid) (PLGA). In an embodiment, the micelle may contain block copolymers. A typical example of the block copolymer is a diblock copolymer. In an embodiment, the diblock copolymer may contain non-polar repeat units and polar repeat units. Typical examples of the polar repeat unit include alkylene glycol (for example, ethylene glycol), alkylene oxide (for example, ethylene oxide), and hydrophilic amino acid. Typical examples of the non-polar repeat unit include γ-L-glutamylglutamine, glutamic acid, lactic acid-co-glycolic acid, phenylene oxide, ε-caprolactone, lactic acid, styrene, butylene oxide, hydrocarbon, and hydrophobic amino acid (for example, aspartic acid). Other block copolymers having more than two different repeat units, for example triblock copolymers, may be used.

In the present invention, the micelle surface preferably has positive charge or negative charge, and thus the micelle may contain a substance having a charge-imparting function, such as an oligopeptide or a derivative thereof. As one aspect of the present invention, in a combination of a capsid with tropism and the micelle, it is preferable that the micelle surface is positively charged when the capsid surface is negatively charged.

In one aspect of the present invention, a nucleic acid is encapsulated inside a micelle. In another aspect of the present invention, nucleic acids modified so as to be amphipathic molecules aggregate to form a micelle. In one aspect of the present invention, peptides can be added to nucleic acids so as to form a micelle. The peptide to be added to nucleic acids may be any peptide as long as it binds to the nucleic acid to form an amphipathic molecule, and a peptide that further gives cell tropism and/or cell permeability to the nanoparticle may be used. The peptide is bound to the nucleic acid, for example, via a linker moiety. An example of the linker moiety is an amide linker. The linker moiety may comprise an optionally substituted piperazinyl moiety, and may further comprise a β-alanine subunit and/or a 6-aminocaproic acid subunit. The peptide can be also bound directly to the nucleic acid without a linker moiety. The peptide may be bound to the nucleic acid at the 3'end or the 5'end.

In the present invention, the nanoparticle may be formed by assembling nucleic acids. In one aspect of the present invention, the nanoparticle is a nanoparticle formed by self-assembly of phosphorodiamidate morpholino oligomers (PMOs) and/or peptide-conjugated PMOs (P-PMOs). The P-PMO is a nucleic acid derivative in which a peptide is bound to a morpholino nucleic acid. The peptide contained in P-PMO may be preferably a peptide having cell membrane permeability, and for example, Pip6a having a sequence of SEQ ID NO: 1 or B peptide having a sequence of SEQ ID NO: 2 can be used. In another aspect of the present invention, the nanoparticle is a nanoparticle formed by self-assembly of tricyclo DNAs (tcDNAs), for example, phosphorothioate tcDNAs. In a further aspect of the present invention, the nanoparticle is a nanoparticle formed by self-assembly of 2'O methyl oligomers (2'OMe).

The complex of the present invention comprises a hollow particle of a non-enveloped virus. In a viral particle (virion), a coat or a shell composed of a plurality of protein units (capsomeres) surrounding a viral nucleic acid or a core is called a capsid. As used herein, when the "capsid" means the structure when the term is simply mentioned. As used herein, the term "hollow particle" means a capsid that does not contain a viral nucleic acid, a core or other substance inside the capsid and is composed of only the capsid proteins. On the other hand, a capsid that contains a viral nucleic acid or a core inside the capsid is called a "nucleocapsid".

The hollow particle may be selected depending on organism species to which the complex of the present invention is administered. For example, when the organism species is an animal, a hollow particle derived from an animal virus may be used, and when the organism species is a plant, a hollow particle derived from a plant virus may be used. It is known that capsids are roughly classified into icosahedral capsids and helical capsids. The hollow particle capsid used for the complex of the present invention may be in any form.

The origin of the hollow particle of a non-enveloped virus used in the present invention is not particularly limited. The non-enveloped virus may be RNA virus or DNA virus. Examples of the hollow particle derived from animal virus that is RNA virus include hollow particles derived from viruses belonging to the family Picornaviridae, the family Caliciviridae, the family Astroviridae, the family Reoviridae, and the family Birnaviridae. Examples of the hollow particle derived from animal virus that is DNA virus include hollow particles derived from viruses belonging to the family Adenoviridae, the family Iridoviridae, the family Circoviridae, the family Parvoviridae, and the family Papovaviridae. Hollow particles derived from viruses belonging to the family Picornaviridae which are RNA viruses and viruses belonging to the family Adenoviridae and the family Parvoviridae which are DNA viruses can be preferably used in the present invention. Hollow particles derived from adenovirus belonging to the family Adenoviridae and AAV belonging to the family Parvoviridae can be particularly preferably used in the present invention.

Examples of hollow particles derived from plant virus that is RNA virus include hollow particles derived from viruses belonging to the genus Tenuivirus, the Tobamovirus group, the family Potyviridae, the Dianthovirus group, the Bromovirus group, the Cucumovirus group, the family Reoviridae, and the Crypticvirus group. Examples of hollow particles derived from plant virus that is DNA virus include hollow particles derived from viruses belonging to the genus Caulimovirus, the genus Badnavirus, and the genus Geminivirus.

The capsomeres constituting the hollow particle capsid may contain mutations as long as they can make up the capsid. As used herein, the "mutation" means that one to several amino acids are replaced, deleted, added or inserted in the amino acid sequence of the capsomere. In the case of amino acid replacement, a replacement between similar amino acids is preferable. The "similar amino acids" refers to amino acids belonging to the same group when amino acids are classified based on properties such as charge, side chain, polarity and aromaticity. Such groups include, for example, a basic amino acid group (arginine, lysine, histidine), an acidic amino acid group (aspartic acid, glutamic acid), a non-polar amino acid group (glycine, alanine, phenylalanine, valine, leucine, isoleucine, proline, methionine, tryptophan), a polar uncharged amino acid group (serine, threonine, asparagine, glutamine, tyrosine, cysteine), a branched amino acid group (leucine, isoleucine, valine), an aromatic amino acid group (phenylalanine, tyrosine), a heterocyclic amino acid group (histidine, tryptophan, proline), and an aliphatic amino acid group (glycine, alanine, leucine, isoleucine, valine). Various mutant capsomeres with tropism for specific cells or tissue are known. Hollow particles containing these mutant capsomeres may be used in the present invention.

The hollow particle used in the present invention may be modified. As used herein, the modification includes a functional modification and a modification as a label. The "functional modification" refers to a modification useful for enhancing or stabilizing specific binding activity between a hollow particle and its target cell. Examples of the functional modification include glycosylation, deglycosylation, and PEGylation of a capsid. The "modification as a label" refers to a modification useful for detecting the complex of the present invention or its target cell in vivo. Examples of the modification as a label include labeling of a capsid with a fluorescent dye [fluorescein, rhodamine, Texas red, Cy3, Cy5, Alexa Fluor (registered trade mark), etc.], a fluorescent protein (for example, PE, APC, GFP, Venus, YFP, DsRed, Sirius, etc.), an enzyme (for example, horseradish peroxidase, alkaline phosphatase, glucose oxidase, etc.), a radioisotope (for example, 3H, 14C, 35S, etc.), and biotin or streptavidin. A method of modifying a capsid is not particularly limited as long as the method allows a protein to be modified and does not affect initial virus infectious activity possessed by the capsid. A commercially available modification kit may be used. Examples of the kit include Alexa Fluor 568 Protein Labeling Kit (A10238) (manufactured by Molecular Probes).

The hollow particle used in the present invention may have a natural capsid or an artificial capsid as long as positive charge or negative charge is distributed on the surface of the capsid so that an ionic bond can be formed. For example, an amino acid is added to the surface of a capsid to adjust zeta potential on the surface of the capsid, and thereby any charge can be distributed on the capsid surface. Further, the any charge is increased or reduced to adjust the Coulomb's force, and thereby the stability of the complex of the present invention can be regulated.

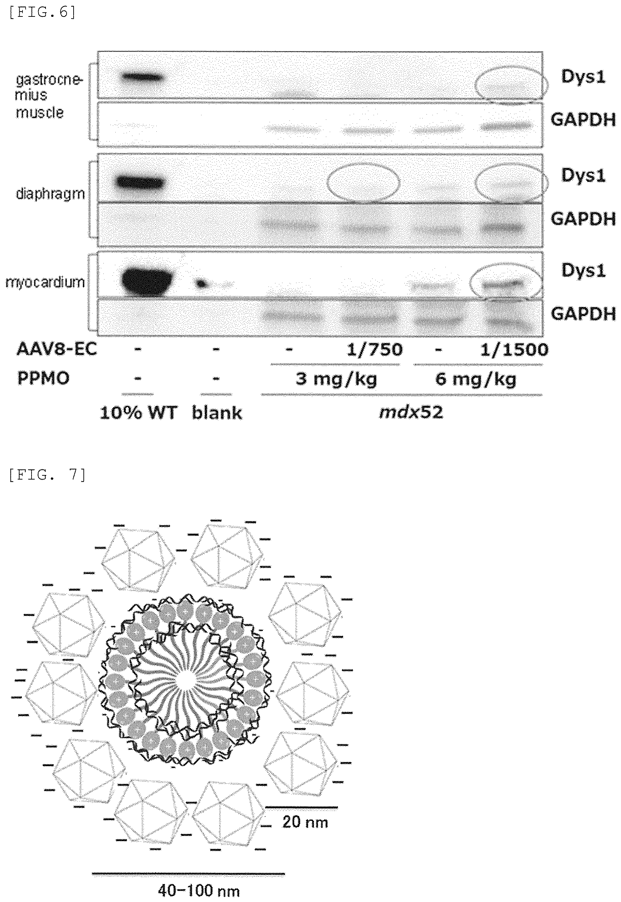

The complex of the present invention comprises a nanoparticle containing a nucleic acid and a hollow particle of a non-enveloped virus, wherein they are bound to each other, for example, electrostatically or chemically (via a covalent bond or an ionic bond) to form the complex. A schematic diagram of the complex of the present invention is shown in Figure 7. However, the schematic diagram of Figure 7 is only an example, and the complex of the present invention is not limited to the form shown in Figure 7. The complex of the present invention may comprise more than one nanoparticle. The present invention also includes the complex comprising two or more nanoparticles and two or more hollow particles, and the complex comprising two or more nanoparticles and one hollow particle.

The size of the complex of the present invention can be determined by transmission electron microscopy (TEM) or a dynamic light scattering (DLS) method, for example, photon correlation spectroscopy, laser diffraction, low-angle laser light scattering (LALS) and medium-angle laser light scattering. (MALLS), or a light obscuration method (for example, Coulter analysis). When the complex of the present invention is measured by TEM, the length of the longest axis (major axis diameter) is for example 50 to 1000 nm, preferably 80 to 800 nm, more preferably 100 to 500 nm. Since the size of the complex of the present invention is distributed within a certain range, the above-mentioned value is the average value or the mode value of the entire complexes.

(2) Method for Producing Complex of the Present Invention

The present invention provides a method for producing the complex of the present invention as described in above (1). The method comprises the following steps:

(i) a step of producing a nanoparticle containing a nucleic acid,
(ii) a step of producing a hollow particle of a non-enveloped virus, and
(iii) a step of mixing the nanoparticle obtained by (i) and the non-enveloped virus hollow particle obtained by (ii) .

In an aspect of the present invention, when a liposome is produced as the nanoparticle, a well-known liposome production method can be used. For example, the liposome can be prepared by solvent injection, lipid hydration, back-evaporation, lyophilization by repeated freezing and thawing. The liposome can be prepared in the form of a multilamellar vesicle or a unilamellar vesicle including a small unilamellar vesicle (SUV) (Methods in Biochemical Analysis, 33: 337, 1988). The liposome containing a nucleic acid can be produced by introducing a nucleic acid into a liposome according to a well-known method.

In another aspect of the present invention, when a micelle is produced as the nanoparticle, a well-known micelle production method can be used. A person skilled in the art understands that many micellar carriers which are amphipathic molecules can self-assemble at critical micelle concentration (cmc) and critical micelle temperature (cmt). The micelle containing a nucleic acid can be produced by mixing micellar carriers and a nucleic acid according to a well-known method. When a nucleic acid is an amphipathic molecule, the micelle can be produced by self-assembling the nucleic acids. For example, a micelle of P-PMO and tcDNA can be produced with reference to Non-Patent Literature 2.

In the production method of the present invention, the hollow particle of a non-enveloped virus can be prepared directly from a host cell infected with the virus from which the hollow particle are derived. For the purpose of obtaining only the hollow particle, it can be also prepared as a recombinant capsid. Preparation methods for such hollow particles are described in Patent Literature 1. Hollow particles are usually formed when a virus propagates in a host cell infected with the virus and then daughter virus particles are constructed. Therefore, the hollow particles can be prepared directly from an extract of a host cell infected with a virus or from a culture medium after release or budding of virus particles. In the extract or culture medium of the host cell, there are not only the hollow particles but also the virus particles. Therefore, it is preferable that the virus particles are separated from the extract or culture medium of the host cell or inactivated to purify the hollow particles. For these purposes, known methods may be used. Examples of a method for separating virus particles and preparing hollow particles include a density gradient centrifugation method using cesium chloride, and a method comprising use of an ion exchange membrane as described in JP-A 2007-11003. In the case where only hollow particles are prepared as a recombinant capsid, an expression vector containing a Cap gene of the desired virus may be expressed in a suitable host cell. For example, for preparing a recombinant hollow particle of AAV, a nucleotide containing a Cap gene of the desired serotype AAV may be inserted into a suitable expression vector. After expressing the Cap gene in a host cell, recombinant hollow particles can be obtained from a cell extract obtained by disrupting the host cell or from a culture medium of the host cell. Collection of the hollow particles from the cell extract or the culture medium may be performed by a method known in the art.

Purification of the AAV-derived hollow particles from a cell homogenate may be performed according to a method known in the art. For example, the hollow particles may be purified by cesium chloride density-gradient ultracentrifugation which is a general method, or by using various types of chromatography including ion exchange chromatography. Further, the hollow particles and the virus particles may be also separated and purified by using an ion exchange membrane.

The step of mixing the nanoparticle and the hollow particle of a non-enveloped virus may further comprise stirring the mixture and/or allowing the mixture to stand. The stirring and the still standing are performed each for 1 to 60 minutes, preferably 5 to 30 minutes. Further, the stirring and the still standing are performed each at 0 to 40°C, preferably 4°C to room temperature.

In the step of mixing the nanoparticle and the hollow particle of a non-enveloped virus, the nucleic acid and the hollow particle are mixed at a molar ratio of nucleic acid to hollow particle of for example 50-10000 : 1, preferably 100-8000 : 1, and more preferably 150-5000 : 1.

The method of the present invention may further comprise a step of adjusting the size of the nanoparticle and/or the complex depending on the intended use. For example, the size adjustment can be performed by passing the nanoparticle and/or the complex through filters having different pore diameters, for example, using an extruder.

(3) Pharmaceutical Composition of the Present Invention

The pharmaceutical composition of the present invention comprises the complex described in above (2) as an active ingredient. An administration route for the pharmaceutical composition is not particularly limited as long as the administration route is pharmaceutically acceptable, and can be selected depending on a treatment method. For example, the pharmaceutical composition of the present invention is preferably administered systemically such as intravenously or intra-arterially, or locally such as intramuscularly, subcutaneously, orally, into tissue, or transdermally. A dosage form for the pharmaceutical composition of the present invention is not particularly limited. Examples of the dosage form include an injection, an oral preparation, a drip infusion, an inhalant, an ointment, and a lotion.

The concentration of the complex of the present invention comprised in the composition of the present invention is suitably in the range of 0.1 nM to 1000 $\mu$M, preferably in the range of 1 nM to 500 $\mu$M, and more preferably in the range of 10 nM to 100 $\mu$M. A dose for in vivo administration of the pharmaceutical composition of the present invention is preferably adjusted considering the type of nucleic acid contained in the complex of the present invention, the dosage form, the patient's conditions such as age and body weight, the administration route, and the nature and extent of disease. In general, for adult humans, the pharmaceutical composition of the present invention is administered in the range of 0.1 mg to 10 g/day, preferably in the range of 1 mg to 1 g/day of the complex of the present invention. The above-mentioned numerical values may vary depending on the type of target disease, the dosage form, and the target molecule. Therefore, in some cases, a dose of less than the above-mentioned numerical values may be sufficient, and in some cases, conversely, a dose of more than the above-mentioned numerical values may be needed. The pharmaceutical composition of the present invention may be administered once to several times a day, or administered repeatedly at an interval of one to several days.

In one aspect of the present invention, the pharmaceutical composition further comprises a pharmaceutically acceptable additive. Examples of the additive include an emulsifying aid (for example, fatty acid with 6 to 22 carbon atoms, or a pharmaceutically acceptable salt thereof, albumin, dextran), a stabilizer (for example, cholesterol, phosphatidic acid), an isotonic agent (for example, sodium chloride, glucose, maltose, lactose, sucrose, trehalose), a pH adjuster (for example, hydrochloric acid, sulfuric acid, phosphoric acid, acetic acid, sodium hydroxide, potassium hydroxide, triethanolamine), and their combinations. The content of the additive in the pharmaceutical composition of the present invention is suitably 90% by weight or less, preferably 70% by weight or less, and more preferably 50% by weight or less.

Diseases to which the pharmaceutical composition of the present invention is applicable are not particularly limited. For various diseases for which effective therapeutic (including symptom relief) or preventive nucleic acid drugs are known, the complexes of the present invention comprising the nucleic acid drugs are prepared, and the complexes can be used as the therapeutic agents or preventive agents. Examples of the applicable diseases include, but not limited to, cancer (solid cancer, blood cancer, etc.), infection, hereditary disease, inflammatory disease, cardiovascular disease, and metabolic syndrome. A subject to which the pharmaceutical composition of the present invention is applicable is not particularly limited. The subject may be a subject to which the nucleic acid drug comprised in the complex of the present invention is applicable or a subject in need of treatment or prevention. Examples of the subject include humans and non-human mammals.

In one aspect of the present invention, the nucleic acid used in the present invention is a nucleic acid (antisense nucleic acid) having a complementary (antisense) nucleotide sequence to a region comprising a splicing-promoting site on the dystrophin gene of a DMD patient (or an animal with DMD). The splicing-promoting site is a region that functions when an intron is excised from a pre-mRNA by splicing. The splicing-promoting site is present in both introns and exons. The antisense nucleic acid inhibits the formation of a spliceosome complex by hybridizing to the splice-promoting site on the pre-mRNA of the dystrophin gene, and induces exon skipping in a sequence-specific manner. Thus, finally, in the splicing process, an exon having a stop codon that causes DMD is skipped and then a mature mRNA that enables the expression of dystrophin protein is produced. Specific examples of the exon that causes DMD include exons 2, 8, 23, 43 to 46, and 50 to 53 in the human dystrophin gene. In one aspect of the present invention, an antisense nucleic acid targeting a sequence of exon 51 as shown in SEQ ID NO: 3 is used.

Whether or not exon skipping of a dystrophin gene occurs can be determined by contacting the complex of the present invention with a dystrophin-expressing cell (for example, a human rhabdomyosarcoma cell), amplifying a region adjacent to a target exon on mRNA of the dystrophin gene from the total RNA of the dystrophin-expressing cell by RT-PCR, and subjecting PCR amplification products to nested PCR or sequence analysis. Skipping efficiency can be determined by measuring "A" that is the polynucleotide amount of the skipped exon in the mRNA of the dystrophin gene and "B" that is the polynucleotide amount of non-skipped exons in the mRNA of the dystrophin gene, and then calculating based on the measurement values of "A" and "B" according to the following formula.

$$\texttt{Skipping efficiency (\%) = A / (A + B) x 100}$$

Preferably, the pharmaceutical composition of the present invention induces exon skipping with an efficiency of 10% or more, 20% or more, 30% or more, 40% or more, 50% or more, 60% or more, 70% or more, 80% or more, or 90% or more.

Examples

Hereinafter, the present invention is more specifically explained with reference to Examples which the scope of the present invention is not limited to.

Example 1: Production and Measurement of Physical Property of AAV Hollow Particle

(1) Production of AAV type 8 and type 9 Hollow Particle

To twenty-eight 225 cm$^2$ flasks (or one 6320 cm$^2$ 10-stage flask) containing 10% FBS$^+$/DMEM/F12 medium, $4 \times 10^8$ 293EB cells as described in WO2012/144446 were inoculated and cultured at 37°C under 5% CO$_2$. After 48 hours of culture, the cells were collected, and an AAV vector plasmid carrying AAV ITRs and an eGFP gene inserted between the ITRs, an AAV helper plasmid carrying rep and cap genes of AAV type 8 (AAV8) or AAV type 9 (AAV9), and an adenovirus helper plasmid carrying E2A, E4 and VA RNA genes of adenovirus type 2 were introduced in an amount of 650 μg each into the cells by a calcium phosphate method. The culture was continued to replicate AAV particles and AAV hollow particles in the cells. The cells were collected by centrifugation 72 hours after the plasmid introduction, and cell pellets were suspended in 30 mL of Tris buffered saline (TBS). Freezing and thawing of a suspension was repeated 4 to 6 times. The suspension was sufficiently mixed by vortex at every thawing.
To the cell suspension after freezing and thawing, 150 μL of 1 M MgCl$_2$ and 20 μL of 250 U/μL Benzonase (manufactured by Merck Millipore) were added. After incubation at 37°C for 30 minutes, 300 μL of 0.5 M EDTA was added to the cell suspension to stop reaction. Then, 900 μL of 5M NaCl was added to the cell suspension, mixed well, and centrifuged at 10,000 × g for 10 minutes at 4°C. A supernatant was collected.
Further, the supernatant thus obtained was heated at 50°C for 30 minutes to denature heat-sensitive protein, and then centrifuged at 4°C and 10,000 × g for 10 minutes. A supernatant was collected.

(2) Crude Purification by Ultracentrifugation

On a 1.50 g/mL cesium chloride solution placed in a centrifuge tube, a 1.25 g/mL cesium chloride solution was layered, and the supernatant collected in above (1) that was previously heat-treated was further layered thereon. After centrifugation at 16°C and 25,000 × g for 3 hours, 0.5 mL fractions of a content fluid were collected from the bottom of the centrifuge tube. The refractive index (RI) of each fraction was measured, and fractions having an RI of 1.365 to 1.368 were mixed. The mixture was dialyzed against about 100 times its volume of an MHN buffer [3.33 mM MES, 3.33 mM HEPES (pH 6.5), 3.33 mM NaOAc] for 30 minutes. The resulting dialysate was diluted with about 5 times its volume of the MHN buffer.

(3) Separation and Purification of Hollow Particle

A cation exchange membrane, Mustang S Acrodisc (manufactured by Pall Corporation) which retains a sulfonic acid group on the surface of a base material as an ion exchange carrier was used. As an FPLC system, AKTA explorer 100 (manufactured by GE Healthcare) was used. Purification was performed as described below. After the Mustang S Acrodisc was equilibrated with the MHN buffer, the diluted solution after dialysis obtained in above (2) was loaded onto the Mustang S Acrodisc at a flow rate of 3 mL/min to adsorb the hollow particles onto the membrane and remove and separate the AAV particles. The Mustang S Acrodisc was washed with 10 CV (10 times the capacity of the disc) of the MHN buffer. The hollow particles were eluted under a concentration gradient condition of 0 to 2 M NaCl/50 CV, and 1 mL fractions were collected. The hollow particles contained in the fractions at the peak absorbance of 280 nm were collected. It was found by electron microscopy that the hollow particles had a black part in the center and did not contain the virus genome.

(4) Measurement of Physical Property of AAV Hollow Particle

The hollow particles of AAV8 and AAV9 prepared in above (1) to (3) were adjusted to be a concentration of 4 $\times$ $10^{13}$ v.p. equivalent/mL with PBS(-) (phosphate buffered saline). The particle size was measured using Viscotek 802 (manufactured by Malvern) by dynamic light scattering (DLS). As a result, the purity of the hollow particles of AAV8 and AAV9 was 96.8% and 98.3%, respectively. The particle size of the hollow particles of both AAV8 and AAV9 was about 28 nm. Further, zeta potential was measured using Zetasizer nano (manufactured by Malvern). The electric conductivity of the hollow particles of both AAV8 and AAV9 was 15 mS/cm, and the isoelectric point of the hollow particles of both AAV8 and AAV9 was around pH 8.5.

Example 2: Production of P-PMO/AAV Hollow Particle Complex (1) Production of P-PMO

Peptide-conjugated phosphorodiamidate morpholino origomers (P-PMO) were prepared according to a method as described in Ezzat K, et al., 2015, NANO LETTERS, vol. 15, pp4364. The P-PMO prepared in this Example consisted of a Pip6a peptide (SEQ ID NO: 1) and a PMO antisense oligonucleotide targeting the sequence set forth in SEQ ID NO: 3 that is a sequence of exon 51 of a mouse dystrophin gene, wherein the PMO antisense oligonucleotide is linked to the C-terminal of Pip6a via an amide linker. A solution of P-PMO was prepared at the concentration of 50 μM with PBS(-).

(2) Production of P-PMO/AAV Hollow Particle Complex

The P-PMO prepared in Example 2 (1) was mixed with the AAV8 hollow particles prepared in Example 1 at a molar ratio of 1500:1, 750:1 or 150:1 in PBS(-) or an Opti-MEM medium (manufactured by Thermo Fisher Scientific), and then allowed to stand at room temperature for 15 minutes.

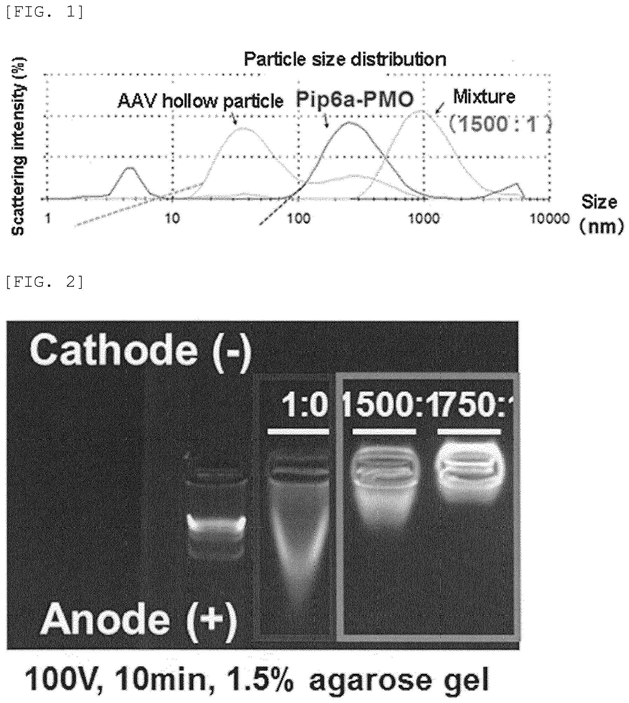

The particle size of substances in the mixture of P-PMO and AAV8 hollow particles at a molar ratio of 1500:1 (50 μM P-PMO, 33.3 nM AAV8 hollow particles) was measured using Zetasizer Nano ZSP by DLS. The particle size distributions of AAV8 hollow particles alone and P-PMO alone were also measured as controls. Measurement results of the particle size distributions by DLS are shown in Figure 1. In addition, the mixtures at molar ratios of 1500:1 and 750:1 were subjected to gel shift assay under the conditions of 1.5% agarose gel, 100 V and 10 minutes. As a control, P-PMO alone was subjected to the gel shift assay. Results of the gel shift assay are shown in Figure 2.

As can be seen from the results shown in Figure 1, the AAV hollow particles, the P-PMO, and the mixture had their respective peak particle sizes. It is known that since hydrodynamic size is measured by DLS, the particle size obtained by DLS is larger than the actual particle size. It has been reported that P-PMO forms micellized nanoparticles having a diameter of about 30 to 90 nm (Non-Patent Literature 2). On the other hand, the particle size of substances contained in the mixture was larger than the particle size of the AAV hollow particles and the particle size of the P-PMO nanoparticles. Thus it was found that a complex of P-PMO/AAV hollow particles was formed in the mixture. As can be also seen from the results of gel shift assay shown in Figure 2, the P-PMO/AAV hollow particle complex was significantly different from P-PMO in electrical property. Furthermore, the P-PMO/AAV hollow particle complex obtained by mixing at a molar ratio of 5000:1 was observed by transmission electron microscope (TEM) imaging. As a result, it was found that there were particles having the longest axis of 100 to 500 nm. When this complex was heat-treated at 95°C for 10 minutes and then subjected to TEM analysis, such a structure was not found.

Example 3: Cell introduction efficiency of P-PMO/AAV Hollow Particle Complex

(1) H2K-mdx52 myoblast

In 0.5 mL of a differentiation medium (a DMEM medium supplemented with 5% horse serum), 50,000 cells of an H2K cell line (H2K-mdx52) derived from an mdx mouse which is a muscular dystrophy model animal (Proc Natl Acad Sci USA 2012 Aug 21; 109 (34): 13763-13768) were cultured for 4 days to differentiate them into myotube cells. Then, the medium was exchanged for an Opti-MEM medium (manufactured by Thermo Fisher Scientific) containing 50 μM (as the concentration of P-PMO) of the P-PMO/AAV hollow particle complex at a molar ratio of 1500:1, 750:1 or 150:1. The cells were cultured for 48 hours without adding a transfection reagent. As a control, the cells were cultured in a medium containing only P-PMO at the same concentration instead of the complex. A total RNA was extracted from the cultured cells using TRIzol (manufactured by Invitrogen). Exons 50 to 53 of the dystrophin gene were amplified by RT-PCR using the RNA as a template and primers Ex50F and Ex53R shown in SEQ ID NO: 4 and SEQ ID NO: 5 respectively.

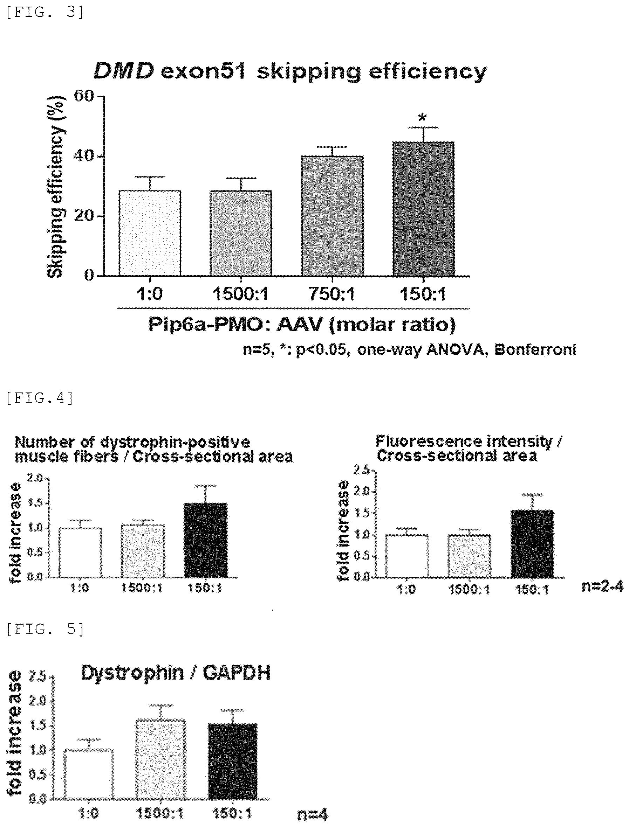

The nucleotide length of an amplified product was analyzed by MultiNA (manufactured by Shimadzu Corporation) to determine exon 51 skipping efficiency. Results are shown in Figure 3. In addition, 15 μL of 0.1% Triton-X100 was added to the cultured cells. The cells were allowed to stand for 30 minutes, suspended by pipetting, and then centrifuged. Lactate dehydrogenase (LDH) in a supernatant was measured as a cell membrane defect marker by using CytoSelect (trademark) LDH cytotoxicity assay kit (manufactured by CBL).

As can be seen from the results shown in Figure 3, the P-PMO/AAV hollow particle complexes showed high exon 51 skipping efficiency as compared to the P-PMO alone. Thus it was shown that the P-PMO/AAV hollow particle complexes had higher cell introduction efficiency than the P-PMO alone. In addition, when the LDH activity was measured as an index of cell membrane defect, no cell membrane defect was found in all of the samples.

(2) mdx52 mouse (local)

To the bilateral tibialis anterior muscles of 5-week-old mdx52 mice (Biochem Biophyss Res Commun. 1997; 238: 492-497) which were raised and bred at the National Center of Neurology and Psychiatry (NCNP), the P-PMO/AAV hollow particle complex at a molar ratio of 1500:1 or 150: 1 was intramuscularly administered in an amount of 10 μg of P-PMO. Two weeks after the administration, a skeletal muscle at the administration site was removed. As a control, a skeletal muscle obtained by administering only P-PMO instead of the complex was used. The skeletal muscles thus obtained were subjected to dystrophin immunohistochemical staining using anti-dystrophin antibody NCL-DYS1 (manufactured by Leica Microsystems), and the number of dystrophin-positive muscle fibers and fluorescence intensity per cross-sectional area of the muscle were measured. Results are shown in Figure 4. Furthermore, proteins were extracted from the removed skeletal muscles with a RIPA buffer supplemented with Complete Mini Protease Inhibitor Cocktail (manufactured by Roche), and subjected to Western blot analysis to determine a relative amount of dystrophin to housekeeping protein GAPDH. Results are shown in Figure 5.

As can be seen from the results shown in Figures 4 and 5, the P-PMO/AAV hollow particle complexes were more effective in enhancing the expression of dystrophin than the P-PMO alone.

(3) mdx52 mouse (systemic)

To 5-week-old mdx52 mice, 3 mg/kg or 6 mg/kg of the P-PMO/AAV hollow particle complex at a molar ratio of 1500:1 or 750:1 was intravenously systematically administered. Two weeks after the administration, a skeletal muscle at each site was removed. As controls, mice that did not receive P-PMO (blank) and mice that received only P-PMO instead of the complex were used. Proteins were extracted from the removed skeletal muscles, and subjected to Western blot analysis in the same manner as in Example 3 (2). Results are shown in Figure 6.

As can be seen from the results shown in Figure 6, the P-PMO/AAV hollow particle complexes highly enhanced the expression of dystrophin in gastrocnemius muscle, diaphragm, and myocardium, as compared to the P-PMO alone.

Sequence Listing Free Text

SEQ ID NO: 1:    Pip6a peptide
SEQ ID NO: 2:    B peptide
SEQ ID NO: 3:    mouse dystrophin gene exon 51 partial sequence
SEQ ID NO: 4:    Ex50F primer
SEQ ID NO: 5:    Ex53R primer

**Claims**

1. A complex comprising a nucleic acid-containing nanoparticle and a hollow particle of a non-enveloped virus.

2. The complex according to claim 1, wherein the nucleic acid is a nucleic acid derivative selected from the group consisting of a phosphorodiamidate morpholino oligomer (PMO), a peptide-conjugated PMO (P-PMO), a tricyclo DNA (tcDNA), and a 2'O methyl oligomer (2'OMe).

3. The complex according to claim 1 or 2, wherein the nucleic acid derivative is a P-PMO containing a peptide having a sequence set forth in SEQ ID NO: 1 or SEQ ID NO: 2.

4. The complex according to any one of claims 1 to 3, wherein the complex has the longest axis of 50 to 1000 nm as measured by a transmission electron microscope (TEM).

5. The complex according to any one of claims 1 to 4, wherein the hollow particle is a hollow particle of an adeno-associated virus.

6. The complex according to any one of claims 1 to 5, wherein the nucleic acid is an antisense nucleic acid complementary to a sequence of a dystrophin gene.

7. The complex according to any one of claims 1 to 6, wherein the nanoparticle is formed by assembly of the nucleic acids.

8. A method for producing a complex comprising a nucleic acid-containing nanoparticle and a capsid virus, the method comprising:

   (i) a step of producing a nanoparticle containing a nucleic acid,

(ii) a step of producing a hollow particle of a non-enveloped virus, and

(iii) a step of mixing the nanoparticle obtained by (i) and the non-enveloped virus hollow particle obtained by (ii) .

9. The method according to claim 8, wherein the nucleic acid and the hollow particle are mixed at a ratio of 150 to 1500 moles of the nucleic acid to 1 mole of the hollow particle in step (iii).

10. A pharmaceutical composition comprising the complex according to any one of claims 1 to 7.

11. The pharmaceutical composition according to claim 10, for use in the treatment of Duchenne muscular dystrophy (DMD).

12. The pharmaceutical composition according to claim 10 or 11, for systemic intravenous administration.

[FIG. 1]

[FIG. 2]

[FIG. 3]

**_DMD_ exon51 skipping efficiency**

n=5, *: p<0.05, one-way ANOVA, Bonferroni

[FIG.4]

**Number of dystrophin-positive muscle fibers / Cross-sectional area**

**Fluorescence intensity / Cross-sectional area**

n=2-4

[FIG. 5]

**Dystrophin / GAPDH**

n=4

[FIG.6]

| | | | | | | |
|---|---|---|---|---|---|---|
| AAV8-EC | - | - | - | 1/750 | - | 1/1500 |
| PPMO | - | - | 3 mg/kg | | 6 mg/kg | |
| | 10% WT | blank | *mdx*52 | | | |

[FIG. 7]

20 nm

40-100 nm

<table>
<tr><td colspan="2" align="center">**INTERNATIONAL SEARCH REPORT**</td><td>International application No.<br>PCT/JP2020/003146</td></tr>
</table>

A.   CLASSIFICATION OF SUBJECT MATTER
A61K   31/7088(2006.01)i;   A61K   31/712(2006.01)i;   A61K   31/7125(2006.01)i;
A61K   48/00(2006.01)i;   A61P   21/04(2006.01)i;   C07K   7/02(2006.01)i;   C07K
14/00(2006.01)i;   B82Y   5/00(2011.01)i;   B82Y   40/00(2011.01)i;   A61K
9/00(2006.01)i;   A61K9/   10(2006.01)i;   A61K   9/14(2006.01)i;   A61K
35/76(2015.01)i;   C12N   7/02(2006.01)i;   A61K   47/64(2017.01)i;   C12N
15/113(2010.01)i
FI:      A61K31/7125;   A61K9/00;   A61K9/10;   A61K9/14;   A61K31/7088;
         A61K31/712 ZNM; A61K35/76; A61K47/64; A61K48/00; A61P21/04 ZNA;
         B82Y5/00; B82Y40/00; C07K7/02; C07K14/00; C12N7/02; C12N15/113 Z
According to International Patent Classification (IPC) or to both national classification and IPC

B.   FIELDS SEARCHED

Minimum documentation searched (classification system followed by classification symbols)
A61K31/7088;   A61K31/712;   A61K31/7125;   A61K48/00;   A61P21/04;   C07K7/02;
C07K14/00;   B82Y5/00;   B82Y40/00;   A61K9/00;   A61K9/10;   A61K9/14;   A61K35/76;
C12N7/02;   A61K47/64;   C12N15/113

Documentation searched other than minimum documentation to the extent that such documents are included in the fields searched
      Published examined utility model applications of Japan          1922–1996
      Published unexamined utility model applications of Japan         1971–2020
      Registered utility model specifications of Japan               1996–2020
      Published registered utility model applications of Japan         1994–2020

Electronic data base consulted during the international search (name of data base and, where practicable, search terms used)
      JSTPlus/JMEDPlus/JST7580 (JDreamIII); CAplus/MEDLINE/EMBASE/BIOSIS (STN);
      Scopus

C.   DOCUMENTS CONSIDERED TO BE RELEVANT

| Category* | Citation of document, with indication, where appropriate, of the relevant passages | Relevant to claim No. |
|---|---|---|
| A | WO 2012/144446 A1 (NATIONAL CENTER OF NEUROLOGY AND PSYCHIATRY) 26.10.2012 (2012-10-26) claims 1, 5-7, 12-14, 17, examples 1-7 | 1-12 |
| A | 伴野太郎 ほか，アデノ随伴ウイルス(AAV)ベクターと AAV 中空粒子，日医大医会誌, 2017, vol. 13, no. 4, pp. 170-171, page 170, right column, lines 5-10, fig. 2, (TOMONO, Taro et al., "Adeno-Associated Virus (AAV) Vector and AAV Empty Particles", Nihon Ika Daigaku Igakkai Zasshi) | 1-12 |

☒   Further documents are listed in the continuation of Box C.          ☒   See patent family annex.

| | |
|---|---|
| *      Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>      18 March 2020 (18.03.2020) | Date of mailing of the international search report<br>      31 March 2020 (31.03.2020) |
|---|---|
| Name and mailing address of the ISA/<br>      Japan Patent Office<br>      3-4-3, Kasumigaseki, Chiyoda-ku,<br>      Tokyo 100-8915, Japan | Authorized officer<br><br><br>Telephone No. |

Form PCT/ISA/210 (second sheet) (January 2015)

**EP 3 936 135 A1**

| | INTERNATIONAL SEARCH REPORT | International application No. |
| | | PCT/JP2020/003146 |

**C (Continuation).  DOCUMENTS CONSIDERED TO BE RELEVANT**

| Category* | Citation of document, with indication, where appropriate, of the relevant passages | Relevant to claim No. |
|---|---|---|
| A | EZZAT, Kariem et al., "Self-Assembly into Nanoparticles Is Essential for Receptor Mediated Uptake of Therapeutic Antisense Oligonucleotides", NANO LETTERS, 2015, vol. 15, pp. 4364-4373, abstract, fig. 1-5 | 1-12 |
| A | JULIANO, R.L., "The delivery of therapeutic oligonucleotides", Nucleic Acids Research, vol. 44, no. 14, 2016, pp. 6518-6548, abstract, fig. 3, section "chemical modification of oligonucleotide", etc. | 1-12 |
| A | MURTHY, V. et al., "Engineering the RNA-Nanobio Interface", Bioengineering, vol. 4, no. 13, 2017, pp. 1-12, doi:10.3390/bioengineering4010013, abstract, fig. 2-4, table 1 | 1-12 |
| A | WO 2015/137409 A1 (NIPPON SHINYAKU CO., LTD.) 17.09.2015 (2015-09-17) claims 1-13, test example 1, fig. 1 | 1-12 |

Form PCT/ISA/210 (continuation of second sheet) (January 2015)

**INTERNATIONAL SEARCH REPORT**
Information on patent family members

International application No.

PCT/JP2020/003146

| Patent Documents referred in the Report | Publication Date | Patent Family | Publication Date |
|---|---|---|---|
| WO 2012/144446 A1 | 26 Oct 2012 | US 2014/0044794 A1 claims 1, 5-7, 12-14, 17, examples 1-7 EP 2700399 A1 | |
| WO 2015/137409 A1 | 17 Sep. 2015 | US 2017/0067048 A1 claims 1-13, examples 1, 2, fig. 1 EP 3118311 A1 KR 10-2016-0132056 A CN 106459955 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012144446 A

- JP 2007011003 A

**Non-patent literature cited in the description**

- **KOMAKI H. et al.** *Science translational medicine,* 2018, vol. 10 (37), eaan0713
- **EZZAT K et al.** *NANO LETTERS,* 2015, vol. 15, 4364
- *Methods in Biochemical Analysis,* 1988, vol. 33, 337

- *Proc Natl Acad Sci USA,* 21 August 2012, vol. 109 (34), 13763-13768
- *Biochem Biophyss Res Commun,* 1997, vol. 238, 492-497